# EUROPEAN PATENT APPLICATION

(11) **EP 4 082 450 A1**
(43) Date of publication of application: **02.11.2022**
(21) Application number: 20905343.8
(22) Date of filing: 04.09.2020
(51) Int. Cl.: A61B 17/12, A61F 2/844, A61F 2/90, A61F 2/07, A61F 2/966

(54) **DISSECTION RUPTURE OCCLUSION SYSTEM**

(30) Priority: 24.12.2019 CN 201911350944
(71) Applicant: Shanghai MicroPort Endovascular MedTech (Group) Co., Ltd., Shanghai 201318 (CN)
(72) Inventor: ZHU, Qing, Shanghai 201318 (CN); TU, Chunlin, Shanghai 201318 (CN); CHEN, Yilu, Shanghai 201318 (CN); ZHANG, Guangjian, Shanghai 201318 (CN); ZHANG, Zhaoduo, Shanghai 201318 (CN); YUAN, Zhenyu, Shanghai 201318 (CN)
(74) Representative: Patentship Patentanwaltsgesellschaft mbH
(86) International application number: PCT/CN2020/113614
(87) International publication number: WO 2021/128939

(57) **Abstract**

A system for occluding a dissection tear, which includes a stent (200) and at least one occluder (100) and is such configured that the stent (200) and the occluder (100) are separate from each other prior to the delivery of the system to a targeted site and connect to each other after the delivery of the system to the targeted site. The stent (200) and the occluder (100) are separately delivered and released in a diseased blood vessel to significantly reduce the resistance encountered during delivery and release of the stent (200) and the occluder (100). Moreover, when introducing the occluder (100) into the false lumen (20), only the profile of the occluder (100) needs to be adjusted, resulting in a lowered surgical difficulty and an improved surgical success rate.

## Description

### TECHNICAL FIELD

The present application relates to the field of medical devices and, more particularly, to a system for occluding a dissection tear.

### BACKGROUND

Aortic dissection (AD) is a condition in which blood flowing in the aortic lumen enters the aortic media from a breach in the aortic intima, and separates expands the tissue of the media apart along the longitudinal direction of the aorta, creating two separated true and false lumens in the aortic wall. AD is an acute, life-threatening cardiovascular disease featuring an abrupt onset, rapid progression and a very high mortality rate. In the current clinical practice, endovascular aortic repair (EVAR) is the most widely used AD therapy. Most AD patients are with multiple tears. However, the principle of the EVAR procedure is to occlude the proximal tear of AD with the implant while leaving distal one(s) untreated, so as to achieve the thrombosis of the false lumen by promoting blood supply in the true lumen and reducing blood flow in the false lumen

Theoretically, in order to achieve better aortic reconstructions and long-term prognosis, it is necessary to treat the distal aortic tears in one procedure or multiple procedures. However, existing interventional treatments of distal aortic dissection tears face the following challenges: (i) distal aortic dissection tears are difficult to treat because they are multiple in number (three or more in most patients) and complex; and (ii) a large proportion of distal aortic dissection tears are present in the abdominal aorta area where branch arteries are present, which makes endovascular treatment difficult. Due to the presence of distal tears, ideal false lumen thrombosis is hard to achieve, and in some treated patients, the false lumens are still open and continues to expand and finally develop into aneurysms.

In the current clinical practice, a common method for promoting false lumen thrombosis is false lumen filling (a procedure to fill a false lumen with an embolization material, which is a spring coil in most cases). Due to the bulky false lumens with many distal tears, the filling requires many spring coils, which may lead to a high surgical difficulty and cost. In addition, the existing spring coil was lack of securing means after released, thereby creating a risk of displacement in the false lumen. In order to overcome this, a therapy using a combination of a stent and a spring coil to occlude tears has been proposed, in which the stent and the spring coil are assembled together to form an integrated occluding system and then delivered to and released in a blood vessel. However, this approach is associated with the following drawbacks: the integrated occluding system encounters great resistance during the release and is difficult to manipulate as the spring coil and the stent need to be adjusted synchronously for entry of the spring coil into a false lumen; and the integrated closure system can occlude only one tear in a certain range and is not able to deal with multiple tears at the same time.

### SUMMARY

It is an object of the present application to provide a system for occluding a dissection tear, which has the advantages of reduced resistances in delivery and release, ease of operation and the ability to deal with multiple tears at the same time.

To this end, the present application provides a system for occluding a dissection tear, comprising a stent and at least one occluder and is configured such that the stent and the occluder are separated from each other prior to a delivery of the system to a targeted site and the stent and the occluder are connected to each other after the delivery of the system to a targeted site.

Optionally, the system for occluding a dissection tear further comprises a first importing device configured to implant the occluder to a targeted site.

Optionally, the first importing device comprises a preloading tube and a push assembly, the preloading tube having a first inner chamber axially extending therethrough, the first inner chamber configured to receive the occluder therein and the preloading tube having a first proximal end and a first distal end opposing the first proximal end, the push assembly having a second proximal end and a second distal end opposing the second proximal end, the second distal end configured to extend into the first proximal end of the first inner chamber and to connect the occluder.

Optionally, the occluder comprises a body and an anchor, the body having a first end and a second end opposing the first end, the anchor being provided at the first end.

When delivering the occluder to a targeted site, the occluder is straightened and received in the first inner chamber, while the anchor is located at the first distal end of the first inner chamber, and the second end of the body is located at the first proximal end of the first inner chamber and connected to the push assembly

Optionally, the occluder comprises a body and an anchor, the body having a first end and a second end opposing the first end, the anchor being provided at the first end.

When delivering the occluder to a targeted site, the occluder is straightened and received in the first inner chamber, while the second end of the body is located at the first distal end of the first inner chamber, and the anchor is located at the first proximal end of the first inner chamber and connected to the push assembly.

Optionally, the first importing device further comprises an importing sheath having a second inner chamber extending axially therethrough, the importing sheath configured to create an importing path for the occluder, and the preloading tube with the occluder loaded therein reaches a targeted site via the second inner chamber.

Optionally, the system for occluding a dissection tear further comprises a second importing device configured to implant the stent to the targeted site.

Optionally, the occluder comprises a body and an anchor, the body comprising a shaping wire and a spring coil, the shaping wire formed by a resilient metal wire and configured to have a predetermined shape, the spring coil sleeved over the shaping wire in order to shape the body with the predetermined shape. The anchor is essentially formed by a resilient metal. The anchor is provided on the body and configured to be connected to the stent.

Optionally, the anchor comprises a positioning portion. The positioning portion is a barb or a securing disc. The stent has a hollow-out structure. The positioning portion is configured to be inserted into the hollow-out structure for connection to the stent.

Optionally, the shaping wire is coiled into a continuous curved structure to form the predetermined shape.

Optionally, the occluder further comprises thrombosis-enhancing villi attached to the body.

Optionally, the stent is fabricated by cutting or braiding. Alternatively, the stent comprises a skeleton covered with a perforated membrane. The hole of the perforated membrane defines the hollow-out structure.

Compared with the prior art, the system for occluding a dissection tear in present application offers the following advantages:
First, the system for occluding a dissection tear includes the stent and the occluder, the system being such configured that the stent and the occluder are separated from each other prior to a delivery of the system to a targeted site and connect to each other after the delivery of the system to a targeted site. That is, the stent and the occluder are coupled to each other only after they have been implanted to the targeted site in the patient's body. In this way, the stent and the occluder can be delivered and released separately to reduce resistance significantly. Moreover, during the entry of the occluder into a false lumen, only the occluder needs to be adjusted, thereby enabling to lower surgical difficulties.

Second, the system for occluding a dissection tear further includes the first importing device including the preloading tube and the push assembly. In this way, depending on how the occluder is loaded in the preloading tube, the occluder may be delivered to the targeted site through either the true or the false lumen, enabling to offer a more flexible use to the system.

### BRIEF DESCRIPTION OF THE DRAWINGS

Fig. 1 is a structural schematic of a system for occluding a dissection tear according to an embodiment of the present application, in which only a stent and a occluder coupled to the stent are shown.
Fig. 2 is a section view of a patient's aorta with aortic dissection (AD).
Fig. 3 schematically illustrates the system according to an embodiment of the present application occluding a dissection tear.
Fig. 4a is a structural schematic of an occluder in a system for occluding a dissection tear according to an embodiment of the present application.
Fig. 4b is a structural schematic of another occluder in a system for occluding a dissection tear according to an embodiment of the present application.
Fig. 5a is a structural schematic of an anchor in an occluder of a system for occluding a dissection tear according to an embodiment of the present application.
Fig. 5b is a structural schematic of another anchor in an occluder of a system for occluding a dissection tear according to an embodiment of the present application.
Fig. 6a is a structural schematic of a stent in a system for occluding a dissection tear according to an embodiment of the present application.
Fig. 6b is a structural schematic of another stent in a system for occluding a dissection tear according to an embodiment of the present application.
Fig. 6c is a structural schematic of still another stent in a system for occluding a dissection tear according to an embodiment of the present application.
Fig. 7 is a structural schematic of a first importing device in a system for occluding a dissection tear according to an embodiment of the present application.
Fig. 8a is a partial cross-section view of the first importing device of Fig. 7, in which the second end of the body is coupled to a push assembly.
Fig. 8b is a partial cross-section view of the first importing device of Fig. 7, in which an anchor is coupled to the push assembly.
Fig. 9 is a structural schematic of a second importing device in a system for occluding a dissection tear according to an embodiment of the present application.
Fig. 10 is a partial cross-section view of the second importing device of Fig. 9.
Figs. 11a to 11e are schematic diagrams illustrating a method for occluding a dissection tear with a system for occluding a dissection tear according to an embodiment of the present application, in which a stent covers only one tear and an occluder is imported to a targeted site through a false lumen.
Fig. 12 schematically illustrates the occlusions of dissection tears using a system according to an embodiment of the present application, in which two tears are occluded by the system.
Figs. 13a to 13e are schematic diagrams illustrating a method for occluding a
dissection tear with a system for occluding a dissection tear according to an embodiment of the present application, in which a stent covers only one tear and an occluder is imported to a targeted site through a true lumen.

In the Figures:
100 - Occluder;
110 - Body;
111 - Ball Head;
120 - Anchor;
121 - Positioning Portion, 122 - Sleeve;
130 - Thrombosis-Enhancing villus;
200 - Stent;
210 - Hollow-out Structure, 220 - Skeleton, 230 - Perforated Membrane;
300 - First Importing Device;
310 - Preloading Tube;
320 - Push assembly;
321 - First Ejection Tube, 322 - Detaching Tube, 323- Connecting Member;
330 - Push Handle;
340 - Detaching Handle;
350 - Importing Sheath;
400 - Second Importing Device;
410 - Conical Tip, 420 - Inner Tube, 430 - Second Ejection Tube, 440 - Outer Sheath, 450 - Securing Handle, 460 - Release Handle; and
10 - True Lumen, 20 - False Lumen, 30 - Tear.

### DETAILED DESCRIPTION

Objects, advantages and features of the present application will become apparent upon reading the following detailed description with reference to the accompanying drawings. It should be noted that the drawings are provided in a very simplified form not necessarily drawn to scale, for the only purpose to facilitate convenient and explicit description of embodiments of the present application.

As used herein, the singular terms "a," "an" and "the" include their plural referents, while the plural form such as the term "plurality" means two or more, unless the context clearly dictates otherwise. As used herein, the term "or" is generally employed in the sense including "and/or", unless the context clearly dictates otherwise, and the terms "attach", "couple" and "connect" in their various forms should be interpreted in a broad sense, which can for example refer to a fixed connection, a detachable connection, or an integral connection, or to a mechanical connection or an electrical connection, or to a direct connection or an indirect connection with one or more elements interposed between the connected ones, or to mutual communication between the interiors of two elements or interaction between two elements. A person of ordinary skilled in the art would be able to understand the specific meanings of these terms in this context based upon specific situations. In the figures, identical or similar reference numerals will be used to identify identical or similar elements.

As used herein, "proximal end" or "distal end" refers to the relative orientation, relative position, or direction of elements or actions that are relative to each other from the perspective of an operator operating the device. Yet without wishing to be limiting in any sense, the "proximal end" generally refers to the end of the medical device close to the operator during its normal operation, while the "distal end" generally refers to the end that enters into the body of the patient first.

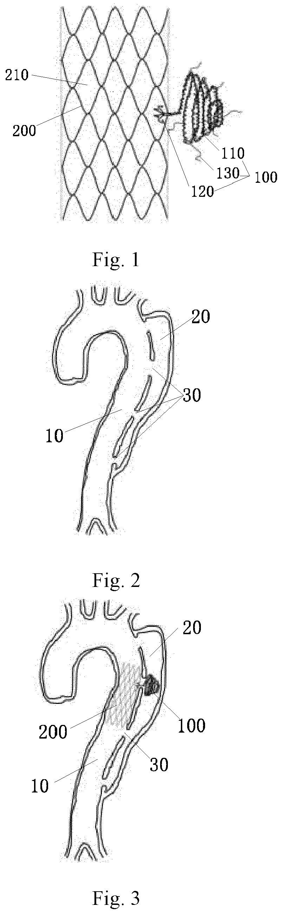

Referring to Fig. 1, an embodiment of the present application provides a system for occluding a dissection tear. The system includes an occluder 100 and a stent 200. At least one occluder 100 may be provided. The system is configured such that the stent 200 is separated from the occluder 100 before the delivery of the system to a targeted site and the stent 200 is connected to the occluder 100 after the delivery of the system to the targeted site.

Reference is now made to Fig. 2, a cross-section view of a patient's aorta with aortic dissection (AD). As shown in Fig. 2, when an AD is occurred in a patient, the aortic intima is torn and separated from the aortic media, which lead to the formation of a true lumen 10 and a false lumen 20 that communicate with each other via the tears 30. In using the system for occluding a dissection tear to treat AD, the stent 200 is delivered into the true lumen 10 first so that the stent 200 supports the true lumen 10 and compresses the false lumen 20. Then, the occluder 100 is delivered to one of the tears 30 so that the occluder 100 fills the false lumen 20 and occludes the tear 30 to block the blood flow into the false lumen 20 and facilitate thrombosis in the false lumen 20. Further, the occluder 100 is coupled to the stent 200 to ensure that the stent 200 can be retained at the tear 30 and any displacement thereof that may deteriorate the closure will not occur under the action of blood flow.

In this embodiment, before the system for occluding a dissection tear is implanted into the diseased blood vessel, the stent 200 and the occluder 100 are separate from each other so that they can be delivered and released in the blood vessel separately. This can effectively reduce resistance encountered during their delivery and release. Moreover, when adjusting the system to enable entry of the occluder 100 into the false lumen 20, only the occluder 100 needs to be adjusted, resulting in a lowered surgical difficulty and an improved surgical success rate.

The specific structure of the system for occluding a dissection tear will be described in greater detail with reference to the accompanying drawings.

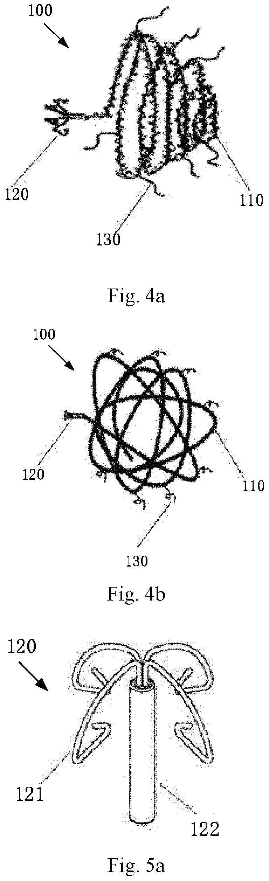

Referring to Fig. 4a, the occluder 100 includes a body 110 and an anchor 120. The body 110 includes a shaping wire (not labeled in the figures) and a spring coil (not labeled in the figures). The shaping wire is made up of a resilient metal wire and configured to have a predetermined shape, and the spring coil is sleeved over the shaping wire to give the body 110 the predetermined shape. The anchor 120 is provided on the body 110 and coupled to the stent 200.

The shaping wire may be made of a shape memory alloy (e.g., a nickel-titanium alloy or the like) or another metal (e.g., a cobalt-chromium alloy, stainless steel, etc.). The shaping wire is coiled into a continuous curved structure to form the predetermined shape. As shown in Fig. 4a, in one embodiment, the shaping wire is helically coiled along a certain axis to form a tapered helix (i.e., the predetermined shape is a tapered helix). The shaping wire is then subjected to a thermal treatment in order to enable the shaping wire 111 to present the predetermined shape in its natural state. When the spring coil is sleeved over the shaping wire, the body 110 also presents as a tapered helix. This arrangement is advantageous in that: firstly, the occluder 100 can be straightened by applying an external force thereon before its delivery into the diseased blood vessel and returns back to the predetermined shape under its own resilience once released in the diseased blood vessel, which avoids insufficient occlusion of the tear due to slackness of the spring coil; secondly, the shaping wire is coiled into a curve to result in the body 110 having no sharp corners, so that the occluder 100 will not cause secondary damages to the tear 30 due to scratching the blood vessel during the release of the occluder 100 in the blood vessel. In some other embodiments, the predetermined shape may be other shapes such as a spherical (in this case, the body 110 is spherical) shape, as shown in Fig. 4b, or a polyhedral (not shown) shape. Further, the body 110 has a first end, where the anchor 120 is provided, and a second end that opposes the first end.

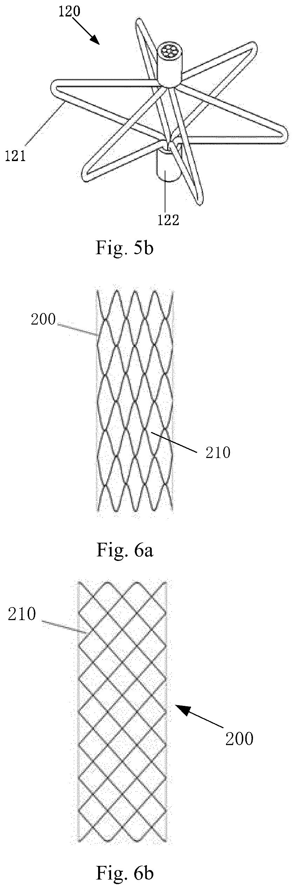

The anchor 120 is resilience and may be made of a shape memory alloy (e.g., a nickel-titanium alloy) or another metal (e.g., a cobalt-chromium alloy, stainless steel or the like). The anchor 120 includes a positioning portion 121. The positioning portion 121 may be a plurality of barbs arranged around an axis, as shown in Fig. 5a, or a securing disc constructed by sequentially arranging a plurality of V-shaped struts around an axis, as shown in Fig. 5b. When a force toward the axis is applied to the positioning portion 121, the positioning portion 121 contracts toward the axis to reduce the volume of anchor 120. Once the force is removed, the positioning portion 121 returns to its original shape.

Further, as shown in Figs. 5a and 5b, the anchor 120 is coupled to the shaping wire. In particular, the anchor 120 further includes a sleeve 122. The positioning portion 121 is fixedly attached to one end of the sleeve 122. The other end of the sleeve 122 is sleeved over the shaping wire. The sleeve 122 is kept stationary axially with respect to the shaping wire while being able to rotate about the axis of the shaping wire. The rotation of the sleeve 122 can be utilized to offset the torsional effect brought by the shaping wire 111 during the release of the occluder 100, so that posture of the anchor 120 can be better adjusted to ensure effective coupling between the anchor 120 and the stent 200.

Optionally, as shown in Figs. 4a and 4b, the occluder 100 further includes thrombosis-enhancing villi 130 that are made of a polymeric material (e.g., PET, PA, PU, PP, etc.) and wound on the body 110. The thrombosis-enhancing villi 130 expand the filling volume of the occluder 100 to improve occluding effect of the body 110 and additionally promote thrombosis in the false lumen 20.

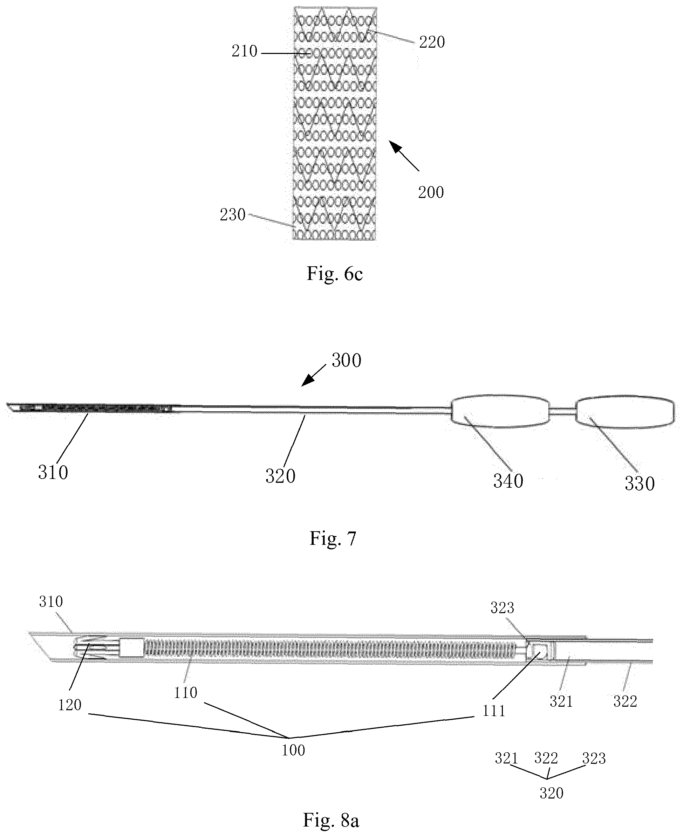

Referring to Figs. 6a to 6c, the stent 200 has a hollow-out structure 210. The stent 200 may be formed in many manners. For example, as shown in Fig. 6a, the stent 200 may have a tubular network structure formed by laser-cutting a tube of a shape memory alloy. In this case, the hole on the stent forms the hollow-out structure 210. The stent 200 may have self-expanding characteristics resulting from a thermal shaping treatment. As another example, as shown in Fig. 6b, the stent 200 may have a tubular network structure formed by weaving filaments. In this case, the hole on the stent forms the hollow-out structure 210. The filaments may be made of a shape memory alloy. Similarly, in this case, the stent 200 may have self-expanding characteristics resulting from a thermal shaping treatment. As yet another example, as shown in Fig. 6c, the stent 200 may be formed by suturing or gluing a perforated membrane 230 on a W-shaped skeleton 220 that is made of a shape memory alloy and shaped by a thermal treatment. In this case, the hole of the perforated membrane 230 forms the hollow-out structure 210.

After implanted into the true lumen 10 of the diseased blood vessel, the self-expanding stent 200 is deployed to continuously support the true lumen 10 and compress the false lumen 20 so as to facilitate thrombosis of the false lumen 20. At the same time, the stent 200 allows blood to flow therethrough and does not affect blood supply to important branch arteries such as the celiac trunk, superior mesenteric artery, renal artery and intercostal artery. In addition, the hollow-out structure 210 of the stent 200 is also configured for the anchor 120 of the occluder 100 passing therethrough to couple the stent 200 and the anchor 120 together. It will be appreciated that, in order to enable effective coupling between the anchor 120 and the stent 200, the size of the hollow-out structure 210 should be smaller than a radial size of the anchor 120 in a natural state and greater than a radial size of the anchor 120 in a constricted state caused by an exerted force, thus allowing the anchor 120 to pass through the hollow-out structure 210.

Further, the system for occluding a dissection tear further includes a first importing device 300 configured to deliver the occluder 100 to a targeted site. Specifically, referring to Figs. 7 and 8a, the first importing device 300 includes a preloading tube 310 and a push assembly 320. The preloading tube 310 has a first inner chamber that axially extends through the tube. The first inner chamber is configured to receive the occluder 100 and has a first proximal end and a first distal end opposing the first proximal end. The push assembly 320 has a second proximal end and a second distal end. The second distal end extends into the first proximal end of the first inner chamber and is configured to connect the occluder 100.

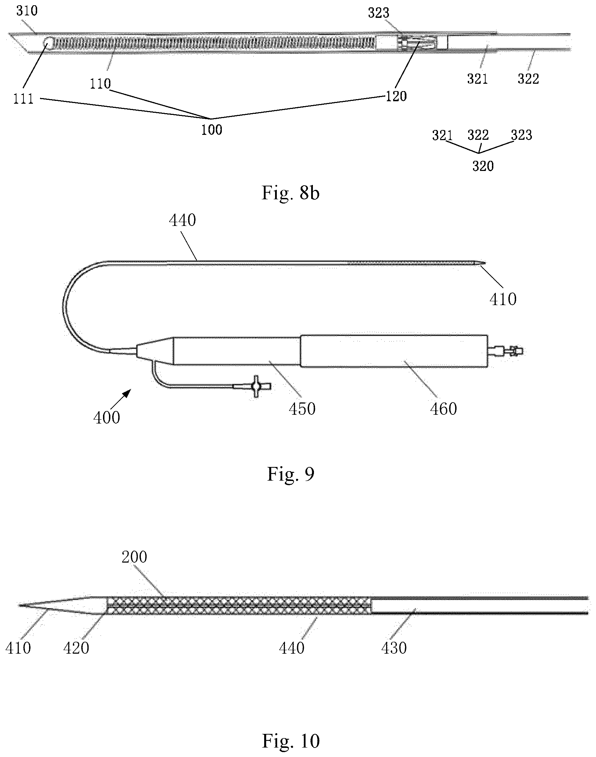

In one embodiment, the push assembly 320 is mechanically coupled to the occluder 100. Specifically, the push assembly 320 includes a first ejection tube 321 and a detaching tube 322. The detaching tube 322 may be hollow and sleeved over the exterior of the first ejection tube 321 so that the detaching tube 322 is axially moveable relative to the first ejection tube 321. The first ejection tube 321 may have a third proximal end and a third distal end opposing the third proximal end, and the detaching tube 322 may have a fourth proximal end and a fourth distal end opposing the fourth proximal end. A connecting member 323 may be provided at the third distal end of the first ejection tube 321 and may be L-shaped so that the axial cross-section between the connecting member 323 and the first ejection tube 321 presents a II-like shape. An opening may be formed in a wall of the connecting member 323 that opposes the end of the first ejection tube 321. The fourth distal end of the detaching tube 322 may be flush with an end of the connecting member 323 away from the first ejection tube 321. In this way, an inner wall of the detaching tube 322, the connecting member 323 and the end portion of the third distal end of the first ejection tube 321 together define a limiting space. Optionally, as shown in Fig. 8a, the occluder 100 is straightened and then loaded in the first inner chamber of the preloading tube 310. Depending on the actual needs, the anchor 120 may be located at a first distal end of the first inner chamber. A ball head 111 may be provided at the second end of the body 110 in the occluder 100, and the coupling between the occluder 100 and the push assembly 320 can be accomplished as the ball head 111 is received and confined in the limiting space. Alternatively, as shown in Fig. 8b, the coupling between the occluder 100 and the push assembly 320 may also be accomplished by arranging the ball head 111 at the first distal end of the first inner chamber and arranging the anchor 120 within the limiting space. As will be described in greater detail below, depending on the orientation of the occluder 100 in the preloading tube 310, the occluder 100 may be delivered to the tear through different paths.

Further, as shown in Fig. 9, the system further includes a first handle assembly including a push handle 330 and a detaching handle 340. The fourth proximal end of the detaching tube 322 may be connected to the detaching handle 340. The third proximal end of the first ejection tube 321 extends along the axial direction of the detaching tube 322 until extending out the detaching handle 340 and then is connected to the push handle 330. In order to release the occluder 100, the first handle assembly may be manipulated to cause both the first ejection tube 321 and the detaching tube 322 to move toward the first distal end so that the occluder 100 is pushed out from the first inner chamber. Upon complete release of the occluder 100, the first handle assembly may be again manipulated to drive the detaching tube 322 or the first ejection tube 321 to move axially so as to push the connecting member 323 out of the fourth distal end of the detaching tube 322, thus removing the coupling between the push assembly 320 and the occluder 100.

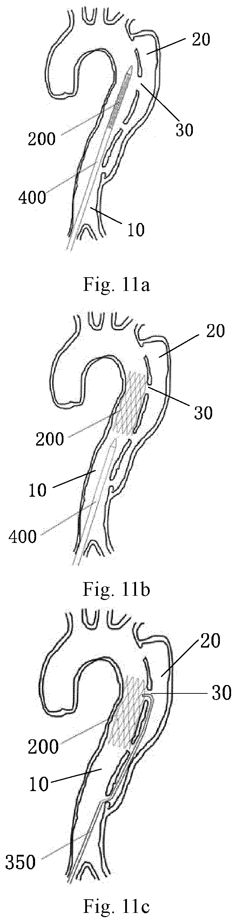

The first importing device 300 may further include an importing sheath 350 (as shown in Fig. 11c) having a second inner chamber extending axially throughout the importing sheath 350. The importing sheath 350 may be configured to create a path through which the occluder 100 can be delivered into the blood vessel. That is, the occluder 100 loaded in the preloading tube 310 enters in the blood vessel through the second inner chamber of the importing sheath 350 and then reaches the tear 30.

The system may further include a second importing device 400 configured to deliver the stent 200 into the true lumen 10. As shown in Figs. 9 and 10, the second importing device 400 includes a conical tip 410, an inner tube 420, a second ejection tube 430 and an outer sheath 440. The inner tube 420 has a fifth proximal end and a fifth distal opposing the fifth proximal end, and the second ejection tube 430 may be hollow and have a sixth proximal end and a sixth distal end opposing the sixth proximal end. The outer sheath 440 may be hollow and have a seventh proximal end and a seventh distal end opposing the seventh proximal end. The inner tube 420 may be nested in the second ejection tube 430 which may be, in turn, nested in the outer sheath 440. The fifth distal end of the inner tube 420 protrudes out of the sixth distal end of the second ejection tube 430 and is then coupled to the conical tip 410, and the seventh distal end of the outer sheath 440 is flush with the fifth distal end of the inner tube 420. In this way, the inner tube 420, the second ejection tube 430 and the outer sheath 440 collectively define an accommodating space. The stent 200 may be disposed over the inner tube 420 and received (in this state, the stent 200 is in a compress state, in which the stent 200 is compressed by the wall of the outer sheath 440) in the accommodating space.

Optionally, the second importing device 400 further includes a second handle assembly including a securing handle 450 and a release handle 460. The securing handle 450 is coupled to both the fifth proximal end of the inner tube 420 and the sixth proximal end of the second ejection tube 430, and the release handle 460 is coupled to the seventh proximal end of the outer sheath 440. The securing handle 450 may be rotatable relative to the release handle 460, and the release handle 460 drives the outer sheath 440 to axially move relative to the inner tube 420 and the second ejection tube 430 by means of a screw transmission mechanism, which enables to release stent 200. It will be appreciated that those skilled in the art will know how to drive the outer sheath 440 to axially move relative to the inner tube 420 and the second ejection tube 430 by the release handle 460, and a detailed description thereof will be omitted herein.

As noted above, the occluder 100 may be loaded in the preloading tube 310 in either of two different manners. According to the different loading manners, there are two different approaches to deliver the system into the diseased blood vessel, as will be detailed below with reference to the accompanying drawings.

In the first approach, when the ball head 111 of the occluder 100 is arranged in the limiting space for coupling to the push assembly 320, the delivering approach for the system includes the following steps.

In step S1, the stent 200 is delivered into the true lumen 10 of the diseased blood vessel by means of the second importing device 400 (as shown in Fig. 11a).

In step S2, the outer sheath 440 is withdrawn to release the stent 200 and cause the stent 200 to cover at least one of the tears 30 (as shown in Fig. 11b).

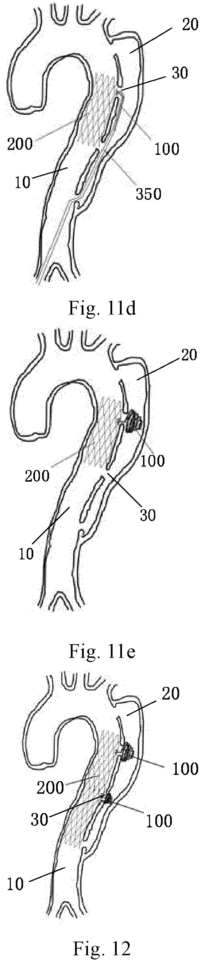

In step S3, the occluder 100 is delivered to the tear 30 by means of the first importing device 300. Specifically, the importing sheath 350 may be advanced to the tear 30 through the false lumen 20 (as shown in Fig. 11c), and the preloading tube 310 in which the occluder 100 is loaded may be delivered to the tear 30 through the second inner chamber of the importing sheath 350 (as shown in Fig. 11d).

In step S4, the push assembly 320 is manipulated to push the occluder 100 so that the anchor 120 is released first, and then passes through the tear 30 and the hollow-out structure 210 on the stent 200 and enters into the stent 200. As a result, anchor 120 is coupled to the stent 200. After that, the occluder 100 is released (as shown in Fig. 11e).

In step S5, the occluder 100 is decoupled from the push assembly 320. Specifically, the first handle assembly is manipulated to drive the detaching tube 322 or the first ejection tube 321 to move axially so that the connecting member 323 protrudes out from the fourth distal end of the detaching tube 322. Consequently, the body 110 can be separated from the push assembly by virtue of the shaping wire's resilience (This is actually a mechanical detaching method).

In other embodiments, the occluder 100 may be coupled to the push assembly 320 in other manners. Depending on the coupling manner, the occluder 100 may be detached from the push assembly 320 electrically (in this case, they may be coupled by a metal wire), by hot melting (in this case, they may be coupled by a polymeric wire), or hydrolytically. It will be appreciated that the electric, hot melt-based and hydrolytic detaching methods are known to those skilled in the art.

Further, the above approach has been described the case in which the system includes only one occluder 100. However, in practice, the system may include two, three or even more occluders 100, depending on the condition of the patient. In the case of the system including two or more occluders 100, the delivering approach for this system remains essentially the same as above, except that steps S3, S4 and S5 are repeated one or more times depending on the number of the occluders 100. Fig. 12 shows a case in which the system includes two occluders 100. In this case, steps S3 and S4 are carried out twice. Preferably, the stent 200 covers all the tears 30, and the number of the occluders 100 is the same as that of the tears 30, so that each tear 30 can be occluded by each occluder 100.

In the second approach, when the anchor 120 of the occluder 100 is arranged in the limiting space for coupling to the push assembly 320, the delivering approach for the system includes the following steps.

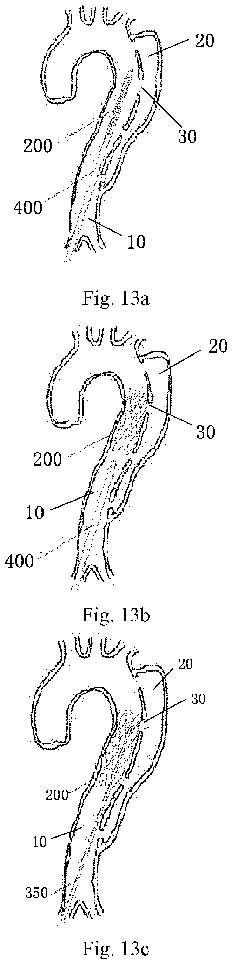

In step S10, the stent 200 is delivered by the second importing device 400 into the true lumen 10 of the diseased blood vessel (as shown in Fig. 13a).

In step S20, the outer sheath 440 is withdrawn to release the stent 200 and cause the stent 200 to cover at least one of the tears 30 (as shown in Fig. 13b).

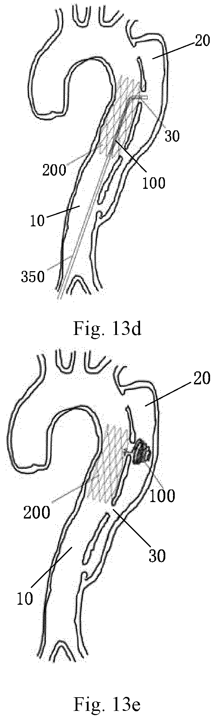

In step S30, the occluder 100 is delivered to the tear 30 by means of the first importing device 300. Specifically, the importing sheath 350 may be advanced to the tear 30 by passing through the true lumen 10, the interior of the stent 200 and the hollow-out strucutre 210 (as shown in Fig. 13c), and then the preloading tube 310 in which the occluder 100 is loaded is delivered to the tear 30 through the second inner chamber of the importing sheath 350 (as shown in Fig. 13d).

In step S40, the push assembly 320 is manipulated to push the occluder 100 so that the second end of the body 110 of the occluder 100 is first released to enable a part of the occluder 100 to pass through the tear 30 and enter in the false lumen 20. After that, the occluder 100 is released continuously until connection between the anchor 120 and the stent 200 is obtained (as shown in Fig. 13e).

At last, depending on the coupling manner between the push assembly 320 and the occluder, the occluder 100 is detached from the push assembly 320 by an appropriate method selected from the mechanical, electrical, hot-melting or hydrolytical method).

Similarly, steps S30 and S40 may be performed one or more times, depending on the number of occluders 100 in the system.

Depending on the actual conditions of AD treatment, the above first and second approaches may be adopted individually or in combination.

The system for occluding a dissection tear proposed in embodiments of the present application comprises a stent and an occluder. The system is such configured that the stent and the occluder are separated from each other prior to a delivery of the system to a targeted site and connect to each other after the delivery of the system to a targeted site. That is, the stent and the occluder are coupled to each other only after they have been implanted to the targeted site in the patient's body. In this way, the stent and the occluder can be delivered and released separately to reduce resistance significantly. Moreover, during the entry of the occluder into a false lumen, only the occluder needs to be adjusted, thereby enabling to lower surgical difficulties. Further, the stent can be used in cooperation with a plurality of such occluders to realize the aim of occluding all tears in a single procedure.

Although the present application has been disclosed above, it is not limited to the above disclosure. Those skilled in the art can make various modifications and variations to the present application without departing from the spirit and scope thereof. Accordingly, the invention is intended to embrace all such modifications and variations if they fall within the scope of the appended claims and equivalents thereof.

## Claims

1. A system for occluding a dissection tear, wherein the system comprises a stent and at least one occluder and is configured such that the stent and the occluder are separated from each other prior to a delivery of the system to a targeted site, and the stent and the occluder are connected to each other after the delivery of the system to the targeted site.

2. The system for occluding a dissection tear of claim 1, further comprising a first importing device configured to implant the occluder to the targeted site.

3. The system for occluding a dissection tear of claim 2, wherein the first importing device comprises a preloading tube and a push assembly, the preloading tube having a first inner chamber axially extending therethrough, the first inner chamber configured to receive the occluder therein, the preloading tube having a first proximal end and a first distal end opposing the first proximal end, the push assembly having a second proximal end and a second distal end opposing the second proximal end, the second distal end configured to extend into the first proximal end of the first inner chamber and to connect the occluder.

4. The system for occluding a dissection tear of claim 3, wherein the occluder comprises a body and an anchor, the body having a first end and a second end opposing the first end, the anchor being provided at the first end, and wherein
when delivering the occluder to the targeted site, the occluder is straightened and received in the first inner chamber, while the anchor is located at the first distal end of the first inner chamber, and the second end of the body is located at the first proximal end of the first inner chamber and connected to the push assembly.

5. The system for occluding a dissection tear of claim 3, wherein the occluder comprises a body and an anchor, the body having a first end and a second end opposing the first end, the anchor being provided at the first end, and wherein
when delivering the occluder to the targeted site, the occluder is straightened and received in the first inner chamber, while the second end of the body is located at the first distal end of the first inner chamber, and the anchor is located at the first proximal end of the first inner chamber and connected to the push assembly.

6. The system for occluding a dissection tear of claim 3, wherein the first importing device further comprises an importing sheath having a second inner chamber extending axially therethrough, the importing sheath configured to create an importing path for the occluder, and wherein the preloading tube with the occluder loaded therein reaches the targeted site via the second inner chamber.

7. The system for occluding a dissection tear of any one of claims 1 to 6, further comprising a second importing device configured to implant the stent at the targeted site.

8. The system for occluding a dissection tear of claim 1, wherein the occluder comprises a body and an anchor, the body comprising a shaping wire and a spring coil, the shaping wire formed by a resilient metal wire and configured to have a predetermined shape, the spring coil sleeved over the shaping wire in order to shape the body with the predetermined shape, wherein the anchor is essentially formed by a resilient metal and wherein the anchor is provided on the body and configured to be connected to the stent.

9. The system for occluding a dissection tear of claim 8, wherein the anchor comprises a positioning portion that is a barb or a positioning disc, wherein the stent has a hollow-out structure, and wherein the positioning portion is configured to be inserted into the hollow-out structure for connecting to the stent.

10. The system for occluding a dissection tear of claim 8, wherein the shaping wire is coiled into a continuous curved structure to form the predetermined shape.

11. The system for occluding a dissection tear of claim 8, wherein the occluder further comprises thrombosis-enhancing villi attached to the body.

12. The system for occluding a dissection tear of claim 9, wherein the stent is fabricated by cutting or braiding, or wherein the stent comprises a skeleton provided with a perforated membrane, a hole of the perforated membrane defining the hollow-out structure.
